(19) **Europäisches Patentamt European Patent Office Office européen des brevets**

(11) **EP 4 497 392 A1**

(12) **EUROPEAN PATENT APPLICATION**

(43) Date of publication:
**29.01.2025  Bulletin 2025/05**

(21) Application number: **23187239.1**

(22) Date of filing: **24.07.2023**

(51) International Patent Classification (IPC):
**A61B 8/08** *(2006.01)*    **A61B 8/00** *(2006.01)*

(52) Cooperative Patent Classification (CPC):
**A61B 8/469; A61B 8/461; A61B 8/52;** A61B 8/08;
A61B 8/0866; A61B 8/0883; A61B 8/463;
A61B 8/5215; A61B 8/5223; G06T 7/0012

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **AIRSANG, Urmila**
  **Eindhoven (NL)**

• **MANIMARAN, Goutham**
  **Eindhoven (NL)**
• **VAJINEPALLI, Pallavi**
  **5656AG Eindhoven (NL)**
• **FIRTION, Celine**
  **Eindhoven (NL)**
• **SRIKRISHNAN, V**
  **Eindhoven (NL)**

(74) Representative: **Philips Intellectual Property & Standards**
**High Tech Campus 52**
**5656 AG Eindhoven (NL)**

(54)  **GENERATING ONE OR MORE INDICATORS OF MOVEMENT**

(57)  A mechanism for generating one or more indicators about a movement of a region of interest through a sequence of ultrasound images. A reference location is defined with respect to the ultrasound images (250). For each ultrasound image, a relative position, direction and/or distance of the region of interest from or with respect to the reference location is identified (230). One or more indicators are produced responsive to the determined position, direction and/or distance of the region of interest (240).

FIG. 2

EP 4 497 392 A1

**Description**

FIELD OF THE INVENTION

The present invention relates to the field of ultrasound imaging, and in particular to tracking the movement of a region of interest during ultrasound imaging.

BACKGROUND OF THE INVENTION

Ultrasound imaging is a popular technique for generating medical image data of a region of interest (ROI) of a medical subject. In particular, ultrasound imaging is one of the most widely used techniques to monitor the growth of a fetus in a womb of a medical subject. Unlike computerized tomography (CT) or magnetic resonance (MR) imaging techniques, which necessitate use of large and cumbersome machinery, ultrasound imaging can be performed using a handheld device. This portability provides significant flexibility of use for ultrasound imaging devices compared to other medical imaging modalities.

However, as ultrasound imaging is typically performed using a handheld device (e.g., a handheld ultrasound transducer), the quality of any resulting ultrasound images will depend heavily upon the manipulation of handheld device by the operator thereof. In particular, a slight deviation of the transducer can cause a major difference in the view plane of the images.

Another problem, from an image quality perspective, in fetal monitoring is the movement of the fetus inside the womb. This movement can cause 'echoes' in any generated ultrasound image or can even result in a completely different view which requires the user to reposition the probe again.

There is therefore an ongoing desire to provide reliable and interpretable information about movement of a region of interest with respect to an ultrasound device. This movement may be due to a movement of the ultrasound device, the subject being monitored or the region of interest within the subject being monitored.

SUMMARY OF THE INVENTION

The invention is defined by the claims.

According to examples in accordance with an aspect of the invention, there is provided a computer-implemented method for outputting one or more indicators for a movement of a region of interest within a sequence of ultrasound images of a medical subject.

The computer-implemented method comprises: obtaining the sequence of ultrasound images of a medical subject; for each ultrasound image in the sequence, processing the ultrasound image to identify the location of a region of interest (ROI) within the ultrasound image, performing a ROI characterization procedure for each ultrasound image in the sequence, the ROI characterization procedure comprising: determining a distance and/or direction between a reference location and the identified location of the region of interest; and/or determining a relative position of the identified location of the region of interest with respect to the reference location; and outputting one or more indicators responsive to the determined distance, direction and/or relative position for each ultrasound image.

The present disclosure thereby provides a mechanism for providing indicators about a movement of a region of interest within a sequence of ultrasound images. In particular, information on the movement with respect to a reference location is output, e.g., for display and/or further processing.

It will be clear that the reference location is a non-origin location of each ultrasound image, i.e., a location that is not positioned at the origin of a Euclidean or Cartesian co-ordinate system defining locations with each ultrasound image. For the avoidance of doubt, it is noted that the reference location is the same for all ultrasound images in the sequence.

In some examples, the ROI characterization procedure comprising determining at least a direction between a reference location and the identified location of the region of interest.

In some examples, the reference location is the average location of the region of interest across the sequence of ultrasound images. This helps provide readily interpretable information about a deviation of the region of interest from an average point, providing more useful information about a stability of the location of the region of interest across the sequence of ultrasound images.

In other examples, the reference location a centroid of the ultrasound image; and/or a location positioned at a predetermined offset from the centroid of the ultrasound image. In general, it is preferred to keep the region of interest at the centroid of the ultrasound image - as this represents the center of insonation during an ultrasound image. By defining the reference location to lie at the centroid of the ultrasound image, deviation from this preferred location can be immediately and readily identified. Use of such reference locations also facilitates real time determination of distances, directions and/or relative locations of locations of the regions of interest with respect to the reference location.

In some examples, for each ultrasound image, the reference location is the average location of the region of interest across the sequence of ultrasound images; and the computer-implemented method further comprises processing the identified location of the region of interest within each ultrasound image to determine the average location of the region of interest across the sequence of ultrasound images.

In some examples, the reference location is a modal average location of the region of interest across the sequence of ultrasound images. This provides an indication of the deviation or movement from a most common position of the region of interest, e.g., to draw closer attention to sudden spikes or shifts of the region of interest.

The ROI characterization procedure may comprise determining a distance between the reference location and the identified location of the region of interest.

In such examples, the step of outputting one or more indicators comprises determining an average distance by processing the determined distance for each ultrasound image in the sequence and outputting a distance indicator responsive to the determined average distance. This provides information on a distance moved by the region of interest, which provides valuable guidance for an operator of the ultrasound imaging system and/or for interpretation of the sequence of ultrasound images (e.g., as this movement may represent a clinically important parameter for consideration).

In some examples, in the ROI characterization process, the step of determining a distance between the reference location and the identified location of the region of interest comprises using the Pythagorean theorem to determine the distance between the reference location and the identified location of the region of interest. This provides an efficient technique for determining the distance between the two locations.

The ROI characterization procedure may comprise determining a direction between the reference location and the identified location of the region of interest.

The step of outputting one or more indicators may comprise determining an average direction by processing the determined direction for each ultrasound image in the sequence and outputting a direction indicator responsive to the determined average direction. The average direction is preferably a mean average direction. This approach helps provide guidance to an operator of the ultrasound imaging system, or reviewer of the sequence of ultrasound images, as to the movement of the ROI, and therefore how the movement should be compensated or taken into account during, for example, further ultrasound imaging and/or analysis of the sequence of ultrasound images.

Optionally, in the ROI characterization process, the step of determining a direction between the reference location and the identified location of the region of interest comprises using a trigonometric process to determine the direction between the reference location and the identified location of the region of interest.

The ROI characterization procedure may comprise determining a relative position of the identified location of the region of interest, with respect to the reference location, in a polar co-ordinate system.

In some examples, the ROI characterization process comprises determining the distance and the direction between the reference location and the identified location of the region of interest; and in the ROI characterization process, the step of determining a relative position of the identified location of the region of interest, with respect to the reference location, in a polar co-ordinate system comprises, processing the determined distance and direction to produce polar co-ordinates of the identified location within the polar co-ordinate system.

In some examples, the identified location of the region of interest and the reference location are defined using Cartesian co-ordinates in a Cartesian co-ordinate system; and in the ROI characterization process, the step of determining a relative position of the identified location of the region of interest, with respect to the reference location, in a polar co-ordinate system comprises, for each dimension of the Cartesian co-ordinate system: producing a quotient by dividing the value of the identified location by the value of the reference location; and multiplying the quotient by a highest possible value for the dimension of the Cartesian co-ordinate system with respect to the ultrasound image.

There is also proposed a computer program product comprising computer program code which, when executed on a computing device having a processing system, cause the processing system to perform all of the steps of any herein disclosed method.

There is also proposed a processing system for outputting one or more indicators for a movement of a region of interest within a sequence of ultrasound images of a medical subject, the processing system being configured to carry out the above method, i.e.: obtain the sequence of ultrasound images of a medical subject; for each ultrasound image in the sequence, process the ultrasound image to identify the location of a region of interest within the ultrasound image, perform an ROI characterization procedure for each ultrasound image in the sequence, the ROI characterization procedure comprising: determining a distance and/or direction between a reference location and the identified location of the region of interest; and/or determining a relative position of the identified location of the region of interest with respect to the reference location; and output one or more indicators responsive to the determined distance, direction and/or relative position for each ultrasound image.

Optionally, for each ultrasound image, the reference location is one of: the average location of the region of interest across the sequence of ultrasound images; a centroid of the ultrasound image; and/or a location positioned at a predetermined offset from the centroid of the ultrasound image.

There is also proposed a user interface system comprising the processing system and a user interface. The processing system is configured to control the user interface to provide a user-perceptible representation (e.g., a display) of the one or more indicators. This approach advantageously provides information to an operator of the ultrasound system and/or an analyzer of the sequence of ultrasound images about the movement of the region of interest.

There is also proposed an ultrasound imaging system comprising an ultrasound transducer configured to generate the sequence of ultrasound images of the medical subject and the processing system herein disclosed. In some examples, the imaging system comprises the user interface system (of which the processing system forms a part).

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1    illustrates an imaging system;
Fig. 2    is a flowchart illustrating a proposed method;
Fig. 3    illustrates a display of indicators;
Fig. 4    illustrates another display of indicators; and
Fig. 5    illustrates a processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating one or more indicators about a movement of a region of interest through a sequence of ultrasound images. A reference location is defined with respect to the ultrasound images. For each ultrasound image, a relative position, direction and/or distance of the region of interest from or with respect to the reference location is identified. One or more indicators are produced responsive to the determined position, direction and/or distance of the region of interest.

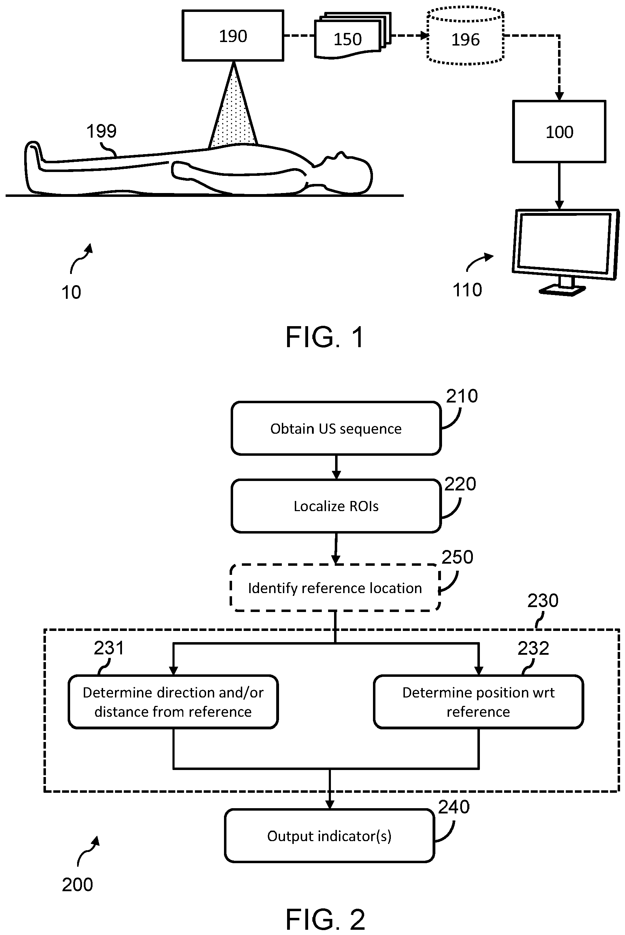

Fig. 1 illustrates an (ultrasound) imaging system 10 in which proposed embodiments can be employed. The imaging system 10 comprises an ultrasound transducer system 190 (e.g., an ultrasound transducer), a processing system 100 and a (optional) user interface 110. The processing system is, by itself, an embodiment of the proposed approach.

The ultrasound transducer system 190 is configured to perform an ultrasound imaging procedure on a medical subject 199 to produce a sequence 150 of ultrasound images. Approaches for performing an ultrasound imaging procedure to produce such a sequence of ultrasound images are well known in the art, and are not detailed for the sake of conciseness. Each ultrasound image is a multidimensional image (e.g., a 2D image or a 3D image) containing a representation of a portion of the medical subject as detected using ultrasound waves.

A sequence of ultrasound images may comprise a temporal sequence, e.g., where each successive ultrasound image is captured or represents a later point/period in time to an earlier ultrasound image in the sequence. However, this is not essential and other forms of sequence will be apparent. For instance, the sequence may be a spatial sequence, e.g., where each successive ultrasound image represents a different location or imaged region.

In the context of the present disclosure, an ultrasound image contains only a representation of the imaged portion of the medical subject. In practice, an ultrasound image may be accompanied by additional information (e.g., scale information, medical subject and so on).

The processing system 100 is configured to output one or more indicators for a movement of a region of interest within the sequence 150 of ultrasound images. The one or more indicators may be used, for instance, to control the user interface 110, e.g., to provide a visual representation of the one or more indicators.

The processing system 100 is configured to obtain the sequence 150 of ultrasound images. The sequence 150 may be obtained directly from the ultrasound transducer system 190 or a database 196 that is itself configured to receive the sequence 150 from the ultrasound transducer system 190. Other approaches for retrieving or obtaining a sequence of ultrasound images will be apparent to the skilled person.

Fig. 2 is a flowchart illustrating a computer-implemented method 200 for outputting the one or more indicators for a movement of a region of interest within the sequence of ultrasound images of the medical subject. The method 200 may, for instance, be performed by the processing system 100.

The method 200 comprises a step 210 of obtaining the sequence of ultrasound images of a medical subject. As previously explained, this may be performed by obtaining the sequence from an ultrasound transducer system and/or a database.

The method 200 also comprises a step 220 of, for each ultrasound image in the sequence, processing the ultrasound image to identify the location of a region of interest within the ultrasound image. In other words, step 220 comprises localizing the region of interest within each ultrasound image.

In step 220, the location of the region of interest may be defined using a set of co-ordinates, e.g., Euclidean or Cartesian co-ordinates. In particular, a single set of co-ordinates (each co-ordinate representing a position along a different dimension) may define the location of the region of interest in a Euclidean space.

The region of interest is the same for each ultrasound image in the sequence, and may, for instance, comprise a particular anatomical structure (e.g., an organ), feature and/or landmark. The precise nature of the region of interest may be application or use-case scenario dependent. For instance, if the sequence of ultrasound images contains a representation of a fetus, then the region of interest may be the fetus. If the sequence of ultrasound images contains a representation of a thorax, then the region of interest may be an anatomical landmark of the heart. Other examples of regions of interest include a femur (e.g., of a fetus), a head (e.g., of a fetus), a growth such as a cancerous growth, an infection, the entrance or exit of a vein or artery, one or more landmarks of the heart, a (kidney) stone, a vertebra and so on.

Approaches for processing an ultrasound image to identify the location of a region of interest (ROI) are widely known, and include any suitable localization technique. One example of a localization technique is a landmark detection technique, which identifies the (single) location of an anatomical landmark. Another example of a localization technique uses a region-identifying technique to identify the bounds or area occupied by the ROI, before defining a location (e.g., a centroid or a corner location) within the bounds or area as the location of the ROI. Examples of region-identifying techniques include image segmentation techniques (e.g., for identifying the bounds of a region of interest) or pixel or pixel region classification techniques (e.g., for identifying pixels containing, and therefore a location of, a region of interest). Appropriate techniques may also be referred to as a localization technique.

A wide variety of suitable techniques for performing localization are known in the art, for instance, as set out by any one or more of the following publications: Noble, J. Alison, and Djarnal Boukerroui. "Ultrasound image segmentation: a survey." IEEE Transactions on medical imaging 25.8 (2006): 987-1010; Wang, Ziyang. "Deep learning in medical ultrasound image segmentation: A review." arXiv preprint arXiv:2002.07703 (2020); Fiorentino, Maria Chiara, et al. "A review on deep-learning algorithms for fetal ultrasound-image analysis." Medical Image Analysis (2022): 102629; Liu, Shengfeng, et al. "Deep learning in medical ultrasound analysis: a review." Engineering 5.2 (2019): 261-275; Meiburger, Kristen M., U. Rajendra Acharya, and Filippo Molinari. "Automated localization and segmentation techniques for B-mode ultrasound images: A review." Computers in biology and medicine 92 (2018): 210-235.

In particular examples, an object detection technique is used to identify the part of the ultrasound image representing a particular object, i.e., a bounding box or contour that is predicted to contain and touch the bounds of the representation of the object in the ultrasound image. Thus, the object may be the region of interest. The location of the object may be identified as a corner or vertex of the identified bounding box or contour.

One suitable object detection technique is the "You only look once" (YOLO) algorithm, as first set out in Redmon, Joseph, et al. "You only look once: Unified, real-time object detection." Proceedings of the IEEE conference on computer vision and pattern recognition. 2016. The YOLO algorithm has undergone further development since this time, e.g., as set out in Jiang, Peiyuan, et al. "A Review of Yolo algorithm developments." Procedia Computer Science 199 (2022): 1066-1073.

Other suitable object detection techniques will be apparent to the skilled person.

Some embodiments for localizing the region of interest may make use of a previously determined location for the region of interest in a previous ultrasound image of the sequence of ultrasound images, e.g., to effectively track the location of the region of interest through the sequence of images. These approaches are well established in the art.

The method also comprises performing an ROI characterization procedure 230 for each ultrasound image in the sequence. The ROI characterization procedure 230 comprises performing a step 231 and/or a step 232. Thus, one or both of steps 231 and 232 may be performed.

Step 231 comprises determining a distance and/or direction between a reference location and the identified location of the region of interest. Step 232 comprises determining a relative position of the identified location of the region of interest with respect to the reference location.

The reference location may, for instance, be the average location of the region of interest across the sequence of ultrasound images; a centroid of the ultrasound image; and/or a location positioned at a predetermined offset from the centroid of the ultrasound image. The reference location is a non-origin location of the ultrasound image.

The average location may, for instance, be a mean average. Approaches for determining a mean average location will be readily apparent to the skilled person. For instance, if the location of a region of interest is defined by an x co-ordinate and a y co-ordinate (e.g., identifying a center or predetermined corner/vertex of the region of interest), then a mean average location can be determined by: averaging (here: summing and then dividing by the total number of ultrasound images) the value of all x co-ordinates to determine an x co-ordinate for the mean average location of the region of interest and averaging (here: summing and then dividing by the total number of ultrasound images) the value of all y co-ordinates to determine an y co-ordinate for the mean average location of the region of interest.

The average location may, for instance, be a modal average location. A modal average location is the location that is most common amongst the locations of all the region(s) of interest across the sequence of ultrasound images.

The method 200 also comprises a step 240 of outputting one or more indicators responsive to the determined distance, direction and/or relative position for each ultrasound image (where relevant). Step 240 may, for instance, comprise controlling a user interface to provide a user-perceptible output (e.g., a display) of the one or more indicators. The user-perceptible output may, for instance be a visual representation of the one or more indicators. In some examples, step 240 comprises storing the indicator(s) in a database or other storage unit, e.g., for later retrieval and/or reference. In yet other examples, step 240 may comprise providing the indicator(s) to a further processing system for undergoing further processing.

The determined indicator(s) are able to effectively act as a quality indicator of the ultrasound sequence, for use by a further processing system or algorithm and/or an operator of the ultrasound transducer system (e.g., to facilitate a decision on whether to repeat the imaging).

In an embodiment, process 230 comprises the step 231 of determining a distance (i.e., a measure of distance) between the reference location and the identified location of the region of interest.

This may be performed using the Pythagorean theorem to determine the (shortest) distance between the reference location and the identified location of the region of interest. Equation (1) defines a suitable approach for determining the distance $D_{pixels(p,q)}$ between the reference location and the identified location in a two-dimensional image:

$$D_{pixels(p,q)} = \sqrt{(q_1 - p_1)^2 + (q_2 - p_2)^2} \qquad (1)$$

where p represents the pixel of the reference location and q represents the pixel for the identified location. Each pixel is represented by a respective pair of co-ordinates, e.g., representing the position along an x and y axis. Thus, pi represents a position along an x axis for the pixel of the reference location, $p_2$ represents a position along a y axis for the pixel of the reference location, $q_1$ represents a position along an x axis for the pixel of the identified location and $q_1$ represents a position along a y axis for the pixel of the identified location.

Equation (1) defines the distance as a measure of pixel distance. It is possible to convert this distance into a true or spatial distance (e.g., measured in cm, mm or inches) by multiplying the distance $D_{pixel(p,q)}$ by a known pixel spacing PS, which represents a true distance between the positions represented by the centroids of two neighboring pixels. Thus, a true distance $D_{true}$ can be calculated using Equation (2):

$$D_{true} = D_{pixels(p,q)} . PS \qquad (2)$$

The above described process effective defines a mechanism for determining a Euclidean distance between the identified location (of the ROI of the ultrasound image) and the reference location. However, any other suitable form of distance measure can be used instead, such as a Manhattan distance or a Minkowski distance.

The distance D may be normalized, e.g., to a maximum possible distance for movement of the region of interest within the ultrasound image, which will be dependent upon the size and/or resolution of the ultrasound image.

In some examples, step 240 comprises determining an average distance by processing the determined distance for each ultrasound image in the sequence and outputting a distance indicator responsive to the determined average distance. Determining an average distance preferably comprises determining a mean distance. In particular, the distance indicator may be the determined average distance itself.

Such embodiments are particularly advantageous when the reference location is the average location (e.g., the modal or mean location) of the region of interest across the sequence of ultrasound images. This is because the average distance will provide useful information on the deviance or movement of the region of interest with respect to a center, which significantly influences an accuracy or interpretability of the region of interest across the sequence of ultrasound images.

In some examples, step 240 comprises determining a weighted average distance by applying a weight (e.g., multiplying the distance by a weight value) to each determined distance for the ultrasound images before determining a mean average of the weighted distances. The distance indicator, responsive to the determined weighted average distance, may then be output.

Applying a weight may comprise increasing a weight value for distances of ultrasound images later in the sequence, e.g., captured at a later point in time. This places greater emphasis on the most recent locations of the region of interest, which will be of more relevance or interest to an operator or further processor. In some instances, the weight value may decrease to zero for distances of ultrasound images earlier in the sequence, effectively creating a time-windowed average of the determined distances.

An average distance, or weighted average distance, finds particular use as a quality indicator or measure of the ultrasound imaging to produce the sequence of ultrasound imaging. Thus, the (weighted) average distance may be output for use by a further processing system or algorithm and/or displayed to an operator of the ultrasound transducer system (e.g., to facilitate a decision on whether to repeat the imaging).

In an embodiment, the ROI characterization procedure comprises determining a direction between the reference location and the identified location of the region of interest. The direction may be defined in terms of an angle from the reference location to the identified location.

This may be performed using a trigonometric process to determine the direction between the reference location and the identified location of the region of interest. Equation (3) illustrates one approach for determining the direction $\theta$ from the reference location to the identified location in a two-dimensional image:

$$\theta = \tan^{-1}\left(\frac{q_2 - p_2}{q_1 - p_1}\right) \qquad (3)$$

where the definitions of pi, $p_2$, $q_1$ and $q_2$ are the same as previously described.

In some examples, step 240 comprises determining an average direction by processing the determined direction for each ultrasound image in the sequence and outputting a direction indicator responsive to the determined average direction. Determining an average direction preferably comprises determining a mean direction. In particular, the direction indicator may be the determined average direction itself.

Such embodiments are particularly advantageous when the reference location is the average location (e.g., the modal or mean location) of the region of interest across the sequence of ultrasound images. This is because the average direction will provide useful information on the deviance or movement of the region of interest with respect to a center, which significantly influences an accuracy or interpretability of the region of interest across the sequence of ultrasound images.

In some examples, step 240 comprises determining a weighted average direction. This may be performed by multiplying each determined distance by a respective weighting value, which is non-negative, to produce weighted determined distances. The sum of the weighted determined distances may then be divided by the sum of the non-negative integer weighting values to generate the weighted average direction. Step 240 may then output the direction indicator responsive to the determined weighted average direction.

Put mathematically, the weighted average direction $\theta_{wav}$ can be determined using the following equation:

$$\theta_{wav} = \frac{\sum_{i=1}^{N} x_i \cdot \theta_i}{\sum_{i=1}^{N} x_i} \qquad (4)$$

where $\theta_i$ is the determined direction for the i-th ultrasound image in the sequence, N is the number of ultrasound images in the sequence, and $x_i$ is the non-negative weighting value for the i-th ultrasound image.

The value of the weighting value $x_i$ may increase for directions of ultrasound images later in the sequence, e.g., ultrasound images captured at a later point in time. This places greater emphasis on the most recent locations of the region of interest, which will be of more relevance or interest to an operator or further processor. In some instances, the weighting value may decrease to zero for distances of ultrasound images earlier in the sequence, effectively creating a time-windowed average of the determined distances.

An average direction, or weighted average direction, finds particular use as a quality indicator or measure of the ultrasound imaging to produce the sequence of ultrasound imaging. Thus, the (weighted) average direction may be output for use by a further processing system or algorithm and/or displayed to an operator of the ultrasound transducer system (e.g., to facilitate a decision on whether to repeat the imaging).

In one embodiment, the ROI characterization procedure comprises determining a relative position of the identified location of the region of interest, with respect to the reference location, in a polar co-ordinate system. Each identified relative position may define a respective indicator to be output or used to control an output.

One approach for determining such a relative position is to determine, for each ultrasound image, a direction and distance of the identified location from the reference location using the techniques previously disclosed . This defines the relative position in the polar co-ordinate system.

Another approach is to directly convert the location of the ROI (e.g., defined as co-ordinates in a Euclidean space) to a position $(x_n, y_n)$ in a polar co-ordinate system. This can be performed using, for example, Equation (5):

$$x_N, y_N = W \times \left(\frac{q_1}{p_1}\right), H \times \left(\frac{q_2}{p_2}\right) \qquad (5)$$

where H and W are the height and width of the ultrasound image respectfully and the definitions of pi, $p_2$, $q_1$ and $q_2$ are the same as previously described.

Thus, in this approach, the step of determining a relative position of the identified location of the region of interest, with respect to the reference location, in a polar co-ordinate system comprises, for each dimension of the Cartesian co-ordinate system: producing a quotient by dividing the value of the identified location by the value of the reference location; and multiplying the quotient by a highest possible value for the dimension of the Cartesian co-ordinate system with respect to the ultrasound image.

In some examples, step 240 comprises controlling a user interface to provide a display of the determined relative positions in the polar co-ordinate system. This provides a useful and readily interpretable map for an operator to understand an amount of movement and a trend or direction of movement. In particular, this display provides information on the direction of any movement of the region of interest.

In some examples, step 240 is configured to define a property of each displayed relative position responsive to a position of the corresponding ultrasound image in the sequence of ultrasound images. For instance, the display of relative positions associated with ultrasound images later in the sequence may be controlled to be more prominently displayed (e.g., greater intensity of display, more opaque, or larger) than the display of relative positions associated with ultrasound images earlier in the sequence. In this way, the display of old points will fade away as new points are made available. Example of suitable properties for a display of a relative position include: opacity; color; intensity; size; shape; pattern and so on.

In some examples, step 240 comprises determining a line of best fit or a trend through the relative positions in the polar co-ordinate system. This line of best fit or trend provides useful information on an amount and/or general direction of movement of the region of interest, to help guide an operator of the ultrasound transducer system to compensate or correct for such movement in future imaging procedures. It will be appreciated that the line of best fit or trend will lie along or define an average direction of the region of interest from the reference point.

In a simple embodiment, the line of best fit may be determined using known or existing plotting techniques, e.g., using the approach put forward by Pearson, Karl. "LIII. On lines and planes of closest fit to systems of points in space." The London, Edinburgh, and Dublin philosophical magazine and journal of science 2.11 (1901): 559-572; or Bland, J. A. "A line of best fit." International Journal of Mathematical Education in Science and Technology 16.5 (1985): 589-592.

In an alternative embodiment, the line of best fit may be determined by using the average direction of the location of interest from the reference location. In particular, the ROI characterization procedure may comprises determining a direction between the reference location and the identified location of the region of interest, e.g., using equation (3). The determined directions may then be averaged (e.g., to determine a mean) in order to determine the average direction of the location of interest from the reference location.

In some examples, the average direction is a weighted average direction, of which approaches for determining the weighted average direction have been previously identified.

In some examples, the length of the line of best fit is responsive to a determined (weighted) average distance, e.g., increases for increasing distances. This provides an intuitive indicator of (weighted) average distance of the region of interest.

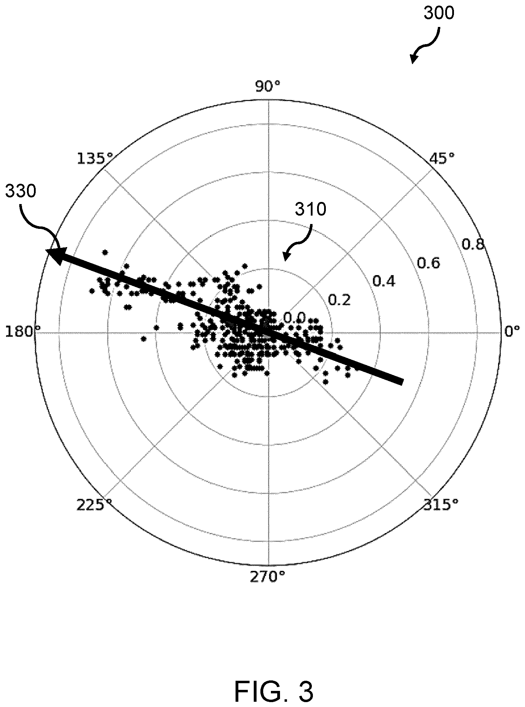

Fig. 3 illustrates an example display 300, by a user interface, produced by a method that carries out an embodiment that determines a relative position of the identified location of the region of interest with respect to the reference location.

The display 300 provides a display of identified locations of a region of interest (with respect to a reference location) defined in a polar co-ordinate system. Thus, there is a cloud 310 of points, each representing a different identified location, about a central location, representing the reference location.

The display 300 also provides a display of a trend or line of best fit 330 of the identified locations, i.e., of the cloud of points. The direction for the trend or line of best fit 330 may have been determined using the (weighted) average direction, e.g., as previously described. The length of the trend or line of best fit 330 may have been determined using the (weighted) average distance (e.g., to increase with increasing (weighted) average distance), or may be predetermined.

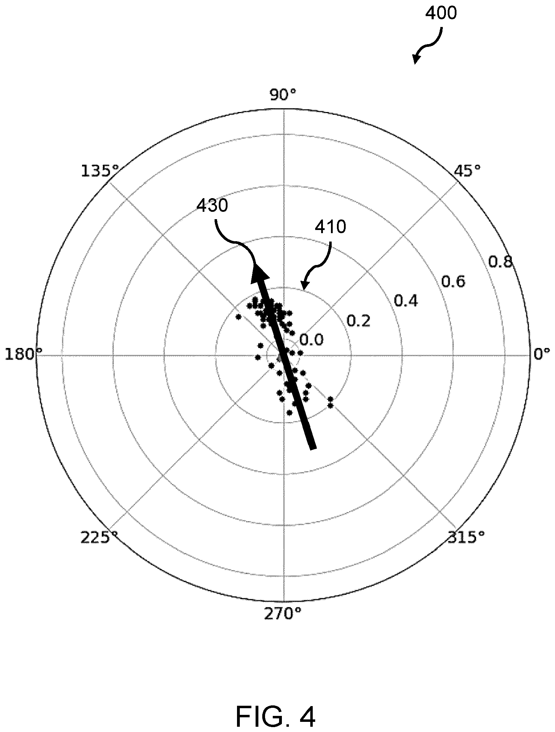

Fig. 4 illustrates another example display 400 by a user interface, produced by a method that carries an embodiment that determines a relative position of the identified location of the region of interest with respect to the reference location. The display again provides an illustration of a cloud 410 of points, each of which represents a different identified location. A center of the display represents the reference location. The display 400 also provides a display of a trend or line of best fit 430 of the identified locations, i.e., of the cloud of points.

As illustrated in Figs 3 and 4, the size of the display of the trend or line of best fit 330, 430 may be dependent upon the average distance between each identified location and the reference location. In particular, the larger the average distance, the larger the display of the trend or line of best fit. Thus, the display may further provide a distance indicator, e.g., calculated according to a previously described approach.

It will be appreciated that, in some examples, the method 200 may perform the functions of any one or more of the previously disclosed embodiment. Thus, step 240 may comprise generating and outputting the distance indicator, the direction indicator and/or the determined relative positions in the polar co-ordinate system.

In previously described examples, a reference location is used in the ROI characterization process. As previously explained, the reference location may be the average location of the region of interest across the sequence of ultrasound images; a centroid of the ultrasound image; and/or a location positioned at a predetermined offset from the centroid of the ultrasound image.

Turning back to Fig. 2, the method 200 may further comprise a step 250 of identifying the reference location. This step may, for instance, be omitted if the reference location is at a known or predetermined location.

In one example, step 250 comprises determining an average location of the region of interest across the sequence of ultrasound images. The average location may be the mean location or the modal location.

In another example, step 250 comprises processing one of the ultrasound images to identify the centroid of a representation of a fanbeam of the ultrasound imaging procedure. The identified centroid of the fanbeam may thereafter act as the reference location.

The proposed method may be performed during or after the ultrasound imaging procedure.

If performed during the ultrasound imaging procedure, it will be appreciated that the method may be iteratively repeated. In such circumstances, it may not be necessary to repeat steps that have already been performed on ultrasound images (in the sequence) that have been previously processed. Rather, the relevant steps of the method may be performed on only those newly obtained ultrasound images in the sequence, which may supplement the output indicators previously provided.

However, in alternative examples, all the steps of the method (preferably except step 220) are repeated each time the sequence of ultrasound images is updated. This may, for instance, be necessary if the reference location is an average location of the region of interest across the sequence of ultrasound images.

Equations (1) to (5) have been described in the context of two-dimensional images. The skilled person would be readily capable of adapting these equations for higher dimensional images (e.g., three-dimensional images) when appropriate.

The skilled person would be readily capable of developing a processing system for carrying out any herein described method. Thus, each step of the flow chart may represent a different action performed by a processing system, and may be performed by a respective module of the processing system.

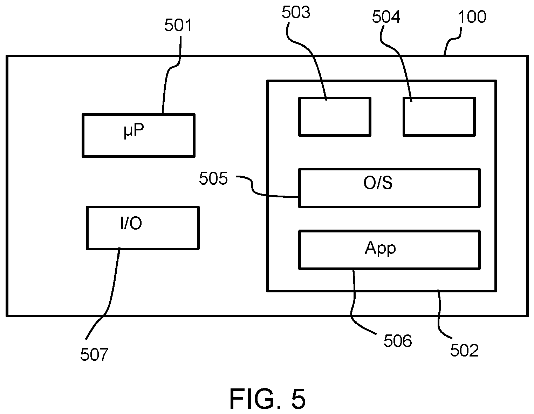

Fig. 5 illustrates one example of a suitable processing system 500.

Various operations discussed above may utilize the capabilities of the computer 500. For example, one or more parts of a processing system for outputting one or more indicators of a movement of a region of interest may be incorporated in any element, module, application, and/or component discussed herein. In this regard, it is to be understood that system functional blocks can run on a single computer or may be distributed over several computers and locations (e.g. connected via the internet).

The computer 500 includes, but is not limited to, PCs, workstations, laptops, PDAs, palm devices, servers, storages, and the like. Generally, in terms of hardware architecture, the computer 500 may include one or more processors 501, memory 502, and one or more I/O devices 507 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 501 is a hardware device for executing software that can be stored in the memory 502. The processor 501 can be virtually any custom made or commercially available processor, a central processing unit (CPU), a digital signal processor (DSP), or an auxiliary processor among several processors associated with the computer 500, and the processor 501 may be a semiconductor based microprocessor (in the form of a microchip) or a microprocessor.

The memory 502 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 502 may incorporate electronic, magnetic, optical, and/or other types of storage media. Note that the memory 502 can have a distributed architecture, where various components are situated remote from one another, but can be accessed by the processor 501.

The software in the memory 502 may include one or more separate programs, each of which comprises an ordered listing of executable instructions for implementing logical functions. The software in the memory 502 includes a suitable operating system (O/S) 505, compiler 504, source code 503, and one or more applications 506 in accordance with exemplary embodiments. As illustrated, the application 506 comprises numerous functional components for implementing the features and operations of the exemplary embodiments. The application 506 of the computer 500 may represent various applications, computational units, logic, functional units, processes, operations, virtual entities, and/or modules in accordance with exemplary embodiments, but the application 506 is not meant to be a limitation.

The operating system 505 controls the execution of other computer programs, and provides scheduling, input-output control, file and data management, memory management, and communication control and related services. It is contemplated by the inventors that the application 506 for implementing exemplary embodiments may be applicable on all commercially available operating systems.

Application 506 may be a source program, executable program (object code), script, or any other entity comprising a set of instructions to be performed. When a source program, then the program is usually translated via a compiler (such as the compiler 504), assembler, interpreter, or the like, which may or may not be included within the memory 502, so as to operate properly in connection with the O/S 505. Furthermore, the application 506 can be written as an object oriented programming language, which has classes of data and methods, or a procedure programming language, which has routines, subroutines, and/or functions, for example but not limited to, Python, C, C++, C#, Pascal, BASIC, API calls, HTML, XHTML, XML, ASP scripts, JavaScript, FORTRAN, COBOL, Perl, Java, ADA, .NET, and the like.

The I/O devices 507 may include input devices such as, for example but not limited to, a mouse, keyboard, scanner, microphone, camera, etc. Furthermore, the I/O devices 507 may also include output devices, for example but not limited to a printer, display, etc. Finally, the I/O devices 507 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 507 also include components for communicating over various networks, such as the Internet or intranet.

If the computer 500 is a PC, workstation, intelligent device or the like, the software in the memory 502 may further include a basic input output system (BIOS) (omitted for simplicity). The BIOS is a set of essential software routines that initialize and test hardware at startup, start the O/S 505, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 501 is configured to execute software stored within the memory 502, to communicate data to and from the memory 502, and to generally control operations of the computer 500 pursuant to the software. The application 506 and the O/S 505 are read, in whole or in part, by the processor 501, perhaps buffered within the processor 501, and then executed.

When the application 506 is implemented in software it should be noted that the application 506 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 506 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method (200) for outputting one or more indicators for a movement of a region of interest within a sequence of ultrasound images (150) of a medical subject (199), the computer-implemented method comprising:

   obtaining (210) the sequence of ultrasound images of a medical subject;
   for each ultrasound image in the sequence, processing (220) the ultrasound image to identify the location of a region of interest, ROI, within the ultrasound image,
   performing a ROI characterization procedure (230) for each ultrasound image in the sequence, the ROI characterization procedure comprising:

   determining (231) a distance and/or direction between a reference location and the identified location of the region of interest; and/or
   determining (232) a relative position of the identified location of the region of interest with respect to the reference location; and

   outputting (240) one or more indicators responsive to the determined distance, direction and/or relative position for each ultrasound image.

2. The computer-implemented method of claim 1, wherein, for each ultrasound image, the reference location is: the average location of the region of interest across the sequence of ultrasound images; a centroid of the ultrasound image; and/or a location positioned at a predetermined offset from the centroid of the ultrasound image.

3. The computer-implemented method of claim 1 or 2, wherein:

   for each ultrasound image, the reference location is the average location of the region of interest across the sequence of ultrasound images; and
   the computer-implemented method further comprises processing the identified location of the region of interest within each ultrasound image to determine the average location of the region of interest across the sequence of ultrasound images.

4. The computer-implemented method of any of claims 1 to 3, wherein the reference location is a modal average location of the region of interest across the sequence of ultrasound images.

5. The computer-implemented method of any of claims 1 to 4, wherein the ROI characterization procedure comprises determining a distance between the reference location and the identified location of the region of interest.

6. The computer-implemented method of claim 5, wherein the step of outputting one or more indicators comprises determining an average distance by processing the determined distance for each ultrasound image in the sequence and outputting a distance indicator responsive to the determined average distance.

7. The computer-implemented method of claim 5 or 6, wherein, in the ROI characterization process, the step of determining a distance between the reference location and the identified location of the region of interest comprises using the Pythagorean theorem to determine the distance between the reference location and the identified location of the region of interest.

8. The computer-implemented method of any of claims 1 to 7, wherein the ROI characterization procedure comprises determining a direction between the reference location and the identified location of the region of interest.

9. The computer-implemented method of claim 8, wherein, in the ROI characterization process, the step of determining a direction between the reference location and the identified location of the region of interest comprises using a trigonometric process to determine the direction between the reference location and the identified location of the region of interest.

10. The computer-implemented method of any of claims 1 to 9, wherein the ROI characterization procedure comprises determining a relative position of the identified location of the region of interest, with respect to the reference location, in a polar co-ordinate system.

11. The computer-implemented method of claim 10 wherein:

the ROI characterization process comprises determining the distance and the direction between the reference location and the identified location of the region of interest; and
in the ROI characterization process, the step of determining a relative position of the identified location of the region of interest, with respect to the reference location, in a polar co-ordinate system comprises, processing the determined distance and direction to produce polar co-ordinates of the identified location within the polar co-ordinate system.

12. The computer-implemented method of claim 10, wherein

the identified location of the region of interest and the reference location are defined using Cartesian co-ordinates in a Cartesian co-ordinate system; and
in the ROI characterization process, the step of determining a relative position of the identified location of the region of interest, with respect to the reference location, in a polar co-ordinate system comprises, for each dimension of the Cartesian co-ordinate system:

producing a quotient by dividing the value of the identified location by the value of the reference location; and
multiplying the quotient by a highest possible value for the dimension of the Cartesian co-ordinate system with respect to the ultrasound image.

13. A computer program product comprising computer program code which, when executed on a computing device having a processing system, cause the processing system to perform all of the steps of the method according to any of claims 1 to 12.

14. A processing system for outputting one or more indicators for a movement of a region of interest within a sequence of ultrasound images of a medical subject, the processing system being configured to perform the method of any of claims 1 to 12.

15. An ultrasound imaging system comprising:

the processing system of claim 14; and
an ultrasound transducer configured to generate the sequence of ultrasound images of the medical subject.

FIG. 1

FIG. 2

FIG. 3

FIG. 4

FIG. 5

**Europäisches Patentamt**
**European Patent Office**
**Office européen des brevets**

# EUROPEAN SEARCH REPORT

Application Number

EP 23 18 7239

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/272543 A1 (KIM NAM-WOONG [KR] ET AL) 1 October 2015 (2015-10-01) | 1,2,5, 7-9, 13-15 | INV. A61B8/08 A61B8/00 |
| Y | * paragraphs [0099] - [0121]; figures 3,7a-c,8 * | 10-12 | |
| X | US 2020/090327 A1 (ROUET JEAN-MICHEL [FR] ET AL) 19 March 2020 (2020-03-19) | 1,8, 13-15 | |
| Y | * paragraphs [0062] - [0085]; figures 5-7 * | 3,4 | |
| X | US 2021/145413 A1 (WEBER FRANK MICHAEL [DE] ET AL) 20 May 2021 (2021-05-20) * paragraphs [0018] - [0151]; figures 1-5 * | 1,2,5,6, 13-15 | |
| Y | JENNE J ET AL: "Ultrasound motion tracking for radiation therapy", RADIOLOGE, DER, SPRINGER, DE, vol. 55, no. 11, 5 October 2015 (2015-10-05), pages 984-991, XP035741851, ISSN: 0033-832X, DOI: 10.1007/S00117-015-0027-0 [retrieved on 2015-10-05] | 3,4 | |
| A | * page 985, middle column, paragraph 3 - page 987, left-hand column, paragraph 1; figure 1 * * page 989, left-hand column, paragraph 1-2 * | 2 | |
| Y | US 5 538 004 A (BAMBER JEFFREY C [US]) 23 July 1996 (1996-07-23) * column 9, line 12 - column 12, line 60; figures 8-9 * | 10-12 | |

**TECHNICAL FIELDS SEARCHED (IPC)**

A61B
G01S

The present search report has been drawn up for all claims

| Place of search | Date of completion of the search | Examiner |
|---|---|---|
| Munich | 8 January 2024 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 7239

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2024

| Patent document cited in search report | | | Publication date | Patent family member(s) | | Publication date |
|---|---|---|---|---|---|---|
| US 2015272543 | A1 | | 01-10-2015 | CN | 104970824 A | 14-10-2015 |
| | | | | EP | 2926737 A1 | 07-10-2015 |
| | | | | KR | 20150114285 A | 12-10-2015 |
| | | | | US | 2015272543 A1 | 01-10-2015 |
| US 2020090327 | A1 | | 19-03-2020 | CN | 110072466 A | 30-07-2019 |
| | | | | EP | 3554379 A1 | 23-10-2019 |
| | | | | JP | 7266523 B2 | 28-04-2023 |
| | | | | JP | 2020503099 A | 30-01-2020 |
| | | | | US | 2020090327 A1 | 19-03-2020 |
| | | | | WO | 2018109114 A1 | 21-06-2018 |
| US 2021145413 | A1 | | 20-05-2021 | CN | 112020331 A | 01-12-2020 |
| | | | | EP | 3569154 A1 | 20-11-2019 |
| | | | | EP | 3781038 A1 | 24-02-2021 |
| | | | | JP | 7346444 B2 | 19-09-2023 |
| | | | | JP | 2021520937 A | 26-08-2021 |
| | | | | US | 2021145413 A1 | 20-05-2021 |
| | | | | WO | 2019201655 A1 | 24-10-2019 |
| US 5538004 | A | | 23-07-1996 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NOBLE** ; **J. ALISON** ; **DJARNAL BOUKERROUI**. Ultrasound image segmentation: a survey. *IEEE Transactions on medical imaging*, 2006, vol. 25 (8), 987-1010
- **WANG, ZIYANG**. Deep learning in medical ultrasound image segmentation: A review. *arXiv preprint arXiv:2002.07703*, 2020
- **FIORENTINO** ; **MARIA CHIARA et al.** A review on deep-learning algorithms for fetal ultrasound-image analysis. *Medical Image Analysis*, 2022, 102629
- **LIU** ; **SHENGFENG et al.** Deep learning in medical ultrasound analysis: a review. *Engineering*, 2019, vol. 5 (2), 261-275
- **MEIBURGER** ; **KRISTEN M.** ; **U. RAJENDRA ACHARYA** ; **FILIPPO MOLINARI**. Automated localization and segmentation techniques for B-mode ultrasound images: A review. *Computers in biology and medicine*, 2018, vol. 92, 210-235
- **REDMON** ; **JOSEPH et al.** You only look once: Unified, real-time object detection. *Proceedings of the IEEE conference on computer vision and pattern recognition*, 2016
- **JIANG** ; **PEIYUAN et al.** A Review of Yolo algorithm developments. *Procedia Computer Science*, 2022, vol. 199, 1066-1073
- **PEARSON** ; **KARL**. LIII. On lines and planes of closest fit to systems of points in space. *The London, Edinburgh, and Dublin philosophical magazine and journal of science*, 1901, vol. 2 (11), 559-572
- **BLAND, J. A**. A line of best fit. *International Journal of Mathematical Education in Science and Technology*, 1985, vol. 16 (5), 589-592